# EUROPEAN PATENT SPECIFICATION

(11) **EP 1 128 827 B1**
(45) Date of publication and mention of the grant of the patent: **02.01.2008**
(21) Application number: 99956029.5
(22) Date of filing: 12.11.1999
(51) Int. Cl.: A61K 31/4439, A61K 9/32, A61K 9/52

(54) **Multilayer tablet comprising rosiglitazone**
Mehrschichtige Tablette, enthaltend Rosiglitazone
Comprimé multicouche comprenant du rosiglitazone

(30) Priority: 12.11.1998 GB 9824870; 25.05.1999 GB 9912189
(43) Date of publication of application: 05.09.2001
(60) Divisional application: 07105991.9
(73) Proprietor: SMITHKLINE BEECHAM PLC, Brentford, Middlesex TW8 9GS (GB); SMITHKLINE BEECHAM CORPORATION, Philadelphia, Pennsylvania 19102 (US)
(72) Inventor: Glinecke, Robert, King of Prussia, PA 19406 (US); Milosovich, Susan Marie, SmithKline Beecham Pharm, Collegeville, PA 19426-0989 (US); Muldoon, William, King of Prussia, PA 19406 (US); Re, Vincenzo, SmithKline Beecham Pharm., Harlow, Essex CM19 5AW (GB); Sauer, Joseph, King of Prussia, PA 19406 (US); Skinner, Janet Louise, SmithKline Beecham Pharm., Harlow, Essex CM19 5AW (GB)
(74) Representative: Rutter, Keith
(86) International application number: PCT/EP1999/009031
(87) International publication number: WO 2000/028990

(56) References cited:
- EP-A- 0 816 340
- EP-A- 0 861 666
- WO-A-98/11884
- WO-A-98/42340
- DE-A- 4 432 757
- US-A- 5 326 770
- US-A- 5 502 078

## Description

This invention relates to a novel composition, in particular to a modified release composition and its use in medicine, especially its use for the treatment of diabetes mellitus, preferably Type 2 diabetes, and conditions associated with diabetes mellitus.

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt at example 1 thereof.

Compound (I) is an example of a class of anti-hyperglycaemic agents known as 'insulin sensitisers'. In particular Compound (I) is a thiazolidinedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

The above mentioned publications are incorporated herein by reference.

It is now indicated that a certain modified release pharmaceutical composition containing Compound (I) allows administration of a single daily dose to maintain a sustained advantageous and beneficial effect upon glycaemic control with mimimal expected adverse side effects. Such a formulation is therefore expected to be particularly useful for the treatment of diabetes mellitus, especially Type 2 diabetes and conditions associated with diabetes mellitus.

Accordingly, the invention provides a pharmaceutical composition suitable for once daily administration, which composition comprises:
a) 2 mg to 12 mg of 5-[4-[2-(N-methyl-N-(2-pyridyl)amino)ethoxy]benzyl]thiazolidine-2,4-dione, optionally in the form of a pharmaceutically acceptable salt, ester or solvate thereof; and
b) a pharmaceutically acceptable carrier therefor;
wherein the composition is a multi-layered tablet arranged to provide a combination of non-modified and sustained release of 5-[4-[2-(N-methyl-N-(2-pyridyl)amino)ethoxy]benzyl]thiazolidine-2,4-dione optionally in the form of a pharmaceutically acceptable salt, ester or solvate thereof, said sustained release being provided by a sustained release matrix selected from disintegrating, non-disintegrating and eroding matrices.

In a further aspect, the invention provides a process for preparing a pharmaceutical composition as described above, which process comprises formulating 5-[4-[2-(N-methyl-N-(2-pyridyl)amino)ethoxy]benzyl]thiazolidine-2,4-dione optionally in the form of a pharmaceutically acceptable salt, ester or solvate thereof and the pharmaceutically acceptable carrier so as to enable a combination of non-modified and sustained release of 5-[4-[2-(N-methyl-N-(2-pyridyl)amino)ethoxy]benzyl]thiazolidine-2,4-dione optionally in the form of a pharmaceutically acceptable salt, ester or solvate thereof.

The sustained release may provide effective release of the active agent over a time period of up to 26 hours, typically in the range of 4 to 24 hours.

Sustained release is suitably obtained by use of a non-disintegrating matrix tablet formulation, for example by incorporating Eudragit RS into the tablet. Alternative non disintegrating matrix tablet formulations are provided by incorporating methacrylates, cellulose acetates, hydroxypropyl methylcellulose phthalate, in particular Eudragit L and RL , Carbopol 971P, HPMCP-HP-55S into the tablet.

Sustained release is further obtained by use of a disintegrating matrix tablet formulation, for example by incorporating methacrylates, methylcellulose, in particular Eudragit L, Methocel K4M into the tablet.

Sustained release can also be achieved by using a multi layer tablet, where the active ingredient is formulated differently in each layer.

Examples of non-modified and sustained release of active agent includes non-modified release of 2mg of Compound (I) and sustained release of 8mg of Compound (I) over a 12 hour period or non-modified release of 2mg of Compound (I) and sustained release of 6mg of Compound (I) over a 12 hour period or non-modified release of 1mg of Compound (I) and sustained release of 7mg of Compound (I) over an 18 hour period. Such controlled release is achieved by use of a multi-layered tablet.

Suitably the composition comprises 2, 3, 4, 5, 6, 7, 8, 9, 10, 11 or 12 mg of Compound (I).

Particularly, the composition comprises 2 to 4 , 4 to 8 or 8 to 12 mg of Compound (I).

Particularly, the composition comprises 2 to 4mg of Compound (I).

Particularly, the composition comprises 4 to 8mg of Compound (I).

Particularly, the composition comprises 8 to 12 mg of Compound (I).

Preferably, the composition comprises 2 mg of Compound (I).

Preferably, the composition comprises 4 mg of Compound (I).

Preferably, the composition comprises 8 mg of Compound (I).

Compound (I) is administered in a pharmaceutically acceptable form, including pharmaceutically acceptable derivatives such as pharmaceutically acceptable salts, esters and solvates thereof, as appropriate.

Suitable pharmaceutically acceptable forms of Compound (I) include those described in EP 0306228 and WO94/05659, especially pharmaceutically acceptable salted forms. A preferred pharmaceutically acceptable salt is a maleate.

Suitable pharmaceutically acceptable solvated forms of Compound (I) include those described in EP 0306228 and WO94/05659, in particular hydrates.

Compound (I) may exist in one of several tautomeric forms, all of which are encompassed by the invention either as individual tautomeric forms or as mixtures thereof. Compound (I) contains one chiral carbon atom, and hence can exist in two stereoisomeric forms; these isomeric forms whether as individual isomers or as mixtures of isomers, including racemates are encompassed by the invention.

Compound (I), or a pharmaceutically acceptable salt thereof, or a pharmaceutically acceptable solvate thereof, may be prepared using known methods, for example those disclosed in EP 0306228 and WO94/05659.

When used herein the term 'conditions associated with diabetes' includes those conditions associated with the pre-diabetic state, conditions associated with diabetes mellitus itself and complications associated with diabetes mellitus.

When used herein the term 'conditions associated with the pre-diabetic state' includes conditions such as insulin resistance, including hereditary insulin resistance, impaired glucose tolerance and hyperinsulinaemia.

'Conditions associated with diabetes mellitus itself include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type 2 diabetes, neuropathy and retinopathy.

Renal diseases associated with Type 2 diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term 'pharmaceutically acceptable' embraces a veterinarily acceptable compound.

For the avoidance of doubt, when reference is made herein to scalar amounts, including mg amounts, of Compound (I) in a pharmaceutically acceptable form, the scalar amount referred to is made in respect of Compound (I) *per se*: For example 2 mg of Compound (I) in the form of the maleate salt is that amount of maleate salt which contains 2 mg of Compound (I).

Diabetes mellitus is preferably Type 2 diabetes.

Glycaemic control may be characterised using conventional methods, for example by measurement of a typically used index of glycaemic control such as fasting plasma glucose or glycosylated haemoglobin (Hb A1c). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

Preferably treatment using the invention will effect an improvement, relative to the non-modified release of the individual agents, in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

The compositions are adapted for oral administration.

The compositions are formulated to provide the modified release of active agent according to the appropriate methods disclosed in Sustained and Controlled Release Drug Delivery Systems, Editor Joe R Robinson, Volume 7, published by Marcel Dekker under the title Drugs and the Pharmaceutical Sciences, Controlled Drug Delivery, 2nd Edition' edited by Joe Robinson and Vince Lee, Marcel Dekker, 1987 and 'Drug Delivery to the Gastrointestinal Tract' Editors: J G Hardy, S S. Davis and C G Wilson also with reference to texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) and Harry's Cosmeticology (Leonard Hill Books).

The tablet may as necessary contain conventional excipients such as binding agents, fillers, lubricants, glidants, disintegrants and wetting agents.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polyvinylpyrrolidone, polacrilin potassium, pregelatinised starch, sodium alginate, sodium lauryl sulphate, sodium starch glycollate.

An example of a pharmaceutically acceptable wetting agent is sodium lauryl sulphate.

As required the solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice.

Compositions may, if desired, be in the form of a pack accompanied by written or printed instructions for use.

No adverse toxicological effects are expected for the compositions of the invention in the above mentioned dosage ranges.

### EXAMPLES

### Example 1, Sustained Release by use of a matrix tablet

A matrix tablet is formed by tabletting the following mixture:

| | |
|---|---|
| | mg/tablet |
| Compound (I) | 8 (pfb) |
| Eudragit L100-55 | 150 |
| Lactose monohydrate | 50 |
| Eudragit RS powder | to 500 |

### Example 2, Sustained Release by use of a Mixed Eudragit matrix tablet

A matrix tablet is formed by tabletting the following mixture:

| | mg/tablet |
|---|---|
| Compound (I) | 8 (pfb) |
| Eudragit L100-55 | 74 |
| Eudragit RS powder | 18.5 |
| Colloidal Silicon dioxide | 0.6 |
| Magnesium stearate | 1.5 |
| Lactose monohydrate | to 150 |

### Example 3, Sustained Release by use of a Mixed Carbopol matrix tablet

A matrix tablet is formed by tabletting the following mixture:

| | mg/tablet |
|---|---|
| Compound (I) | 8 (pfb) |
| Anhydrous dibasic calcium phosphate | 35.7 |
| Carbopol 971P | 22.5 |
| Carbopol 974P | 7.5 |
| Talc | 0.75 |
| Lactose monohydrate | to 150 |

## Claims

1. A pharmaceutical composition suitable for once daily administration, which composition comprises:
a) 2 mg to 12 mg of 5-[4-[2-(N-methyl-N-(2-pyridyl)amino)ethoxy]benzyl]thiazolidine-2,4-dione, optionally in the form of a pharmaceutically acceptable salt, ester or solvate thereof; and
b) a pharmaceutically acceptable carrier therefor;
wherein the composition is a multi-layered tablet arranged to provide a combination of non-modified and sustained release of 5-[4-[2-(N-methyl-N-(2-pyridyl)amino)ethoxy]benzyl]thiazolidine-2,4-dione optionally in the form of a pharmaceutically acceptable salt, ester or solvate thereof, said sustained release being provided by a sustained release matrix selected from disintegrating, non-disintegrating and eroding matrices.

2. A composition according to claim 1 wherein the disintegrating matrix tablet formulation is provided by incorporating methacrylates, methylcellulose and Methocel K4M into the matrix.

3. A composition according to claim 1, wherein the non-disintegrating matrix tablet formulation is provided by incorporating Eudragit RS, methacrylates, cellulose acetates, hydroxypropyl methylcellulose phthalate, Carbopol 971P or HPMCP-HP-55S into the matrix.

4. A composition according to any preceding claim, wherein 5-[4-[2-(N-methyl-N-(2-pyridyl)amino)ethoxy]benzyl]thiazolidine-2,4-dione is in the form of a maleate salt.

5. A process for preparing a pharmaceutical composition according to claim 1, which process comprises formulating 5-[4-[2-(N-methyl-N-(2-pyridyl)amino)ethoxy]benzyl]thiazolidine-2,4-dione optionally in the form of a pharmaceutically acceptable salt, ester or solvate thereof and the pharmaceutically acceptable carrier so as to enable a combination of non-modified and sustained release of 5-[4-[2-(N-methyl-N-(2-pyridyl)amino)ethoxy]benzyl]thiazolidine-2,4-dione optionally in the form of a pharmaceutically acceptable salt, ester or solvate thereof.

## Patentansprüche

1. Arzneimittelzusammensetzung, geeignet zur einmal täglichen Verabreichung, wobei die Zusammensetzung umfasst:
a) 2 mg bis 12 mg 5-[4-[2-(N-Methyl-N-(2-pyridyl)amino)ethoxy]benzyl]thiazolidin-2,4-dion, gegebenenfalls in Form eines pharmazeutisch verträglichen Salzes, Esters oder Solvats davon; und
b) einen pharmazeutisch verträglichen Träger dafür;
wobei die Zusammensetzung eine mehrschichtige Tablette ist, die aufgebaut ist, um eine Kombination von nicht-modifizierter und verlängerter Freisetzung von 5-[4-[2-(N-Methyl-N-(2-pyridyl)amino)ethoxy]benzyl]thiazolidin-2,4-dion, gegebenenfalls in Form eines pharmazeutisch verträglichen Salzes, Esters oder Solvats davon, bereitzustellen, wobei die verlängerte Freisetzung durch eine Matrix zur verlängerten Freisetzung, ausgewählt aus sich zersetzenden, sich nicht zersetzenden und zerfallenden Matrices, bereitgestellt wird.

2. Zusammensetzung nach Anspruch 1, wobei die Tablettenformierung mit sich zersetzender Matrix durch Aufnehmen von Methacrylaten, Methylcellulose und Methocel K4M in die Matrix bereitgestellt wird.

3. Zusammensetzung nach Anspruch 1, wobei die Tablettenformulierung mit sich nicht zersetzender Matrix durch Aufnehmen von Eudragit RS, Methacrylaten, Celluloseacetaten, Hydroxypropylmethylcellulosephthalat, Carbopol 971P oder HPMCP-HP-55S in die Matrix bereitgestel lt wird.

4. Zusammensetzung nach einem vorhergehenden Anspruch, wobei 5-[4-[2-(N-Methyl-N-(2-pyridyl)amino)ethoxy]benzyl]thiazolidin-2,4-dion in Form eines Maleatsalzes vorliegt.

5. Verfahren zur Herstellung einer Arzneimittelzusammensetzung nach Anspruch 1, wobei das Verfahren das Formulieren von 5-[4-[2-(N-Methyl-N-(2-pyridyl)amino)ethoxy]benzyl]thiazolidin-2,4-dion, gegebenenfalls in Form eines pharmazeutisch verträglichen Salzes, Esters oder Solvats davon, und des pharmazeutisch verträglichen Trägers derart umfasst, dass eine Kombination von nicht-modifizierter und verlängerter Freisetzung von 5-[4-[2-(N-Methyl-N-(2-pyridyl)amino)-ethoxy]benzyl]thiazolidin-2,4-dion, gegebenenfalls in Form eines pharmazeutisch verträglichen Salzes, Esters oder Solvats davon, ermöglicht wird.

## Revendications

1. Composition pharmaceutique convenable pour une administration une fois par jour, composition qui comprend :
a) 2 mg à 12 mg de 5-[4-[2-(N-méthyl-N-(2-pyridyl)-amino)éthoxy]benzyl]thiazolidine-2,4-dione, éventuellement sous forme d'un de ses sels, esters ou produits de solvatation pharmaceutiquement acceptables ; et
b) un support pharmaceutiquement acceptable à cette fin ;
la composition étant un comprimé multicouche conçu pour fournir une association de libération non modifiée et de libération prolongée de 5-[4-[2-(N-méthyl-N-(2-pyridyl)-amino)éthoxy]benzyl]thiazolidine-2,4-dione éventuellement sous forme d'un de ses sels, esters ou produits de solvatation pharmaceutiquement acceptables, ladite libération prolongée étant fournie par une matrice à libération prolongée choisie entre des matrices à délitement, des matrices sans délitement et des matrices à érosion.

2. Composition suivant la revendication 1, dans laquelle la formulation de comprimé à matrice à délitement est fournie en incorporant des méthacrylates, de la méthylcellulose et du Méthocel K4M à la matrice.

3. Composition suivant la revendication 1, dans laquelle la formulation de comprimé à matrice sans délitement est fournie en incorporant de l'Eudragit RS, des méthacrylates, des acétates de cellulose, du phtalate d'hydroxypropylméthylcellulose, du Carbopol 971P ou de la HPMCP-HP-55S à la matrice.

4. Composition suivant l'une quelconque des revendications précédentes, dans laquelle la 5-[4-[2-(N-méthyl-N-(2-pyridyl)amino)éthoxy]benzyl]thiazolidine-2,4-dione est sous forme d'un sel maléate.

5. Procédé pour la préparation d'une composition pharmaceutique suivant la revendication 1, procédé qui comprend la formulation de 5-[4-[2-(N-méthyl-N-(2-pyridyl)-amino)éthoxy]benzyl]thiazolidine-2,4-dione éventuellement sous forme d'un de ses sels, esters ou produits de solvatation pharmaceutiquement acceptables et du support pharmaceutiquement acceptable de manière à permettre une association de libération non modifiée et de libération prolongée de 5-[4-[2-(N-méthyl-N-(2-pyridyl)amino)éthoxy]-benzyl]thiazolidine-2,4-dione, éventuellement sous forme d'un de ses sels, esters ou produits de solvatation pharmaceutiquement acceptables.
